# EUROPEAN PATENT SPECIFICATION

(11) **EP 3 369 443 B1**
(45) Date of publication and mention of the grant of the patent: **08.06.2022**
(21) Application number: 16859959.5
(22) Date of filing: 28.10.2016
(51) Int. Cl.: A61M 1/36

(54) **BLOOD PURIFICATION APPARATUS**
BLUTREINIGUNGSVORRICHTUNG
DISPOSITIF DE PURIFICATION SANGUINE

(30) Priority: 30.10.2015 JP 2015214220
(43) Date of publication of application: 05.09.2018
(73) Proprietor: Nikkiso Company Limited, Tokyo 150-6022 (JP)
(72) Inventor: MOCHIZUKI, Hiroaki, Makinohara-shi Shizuoka 421-0496 (JP); MATSUO, Sumiaki, Makinohara-shi Shizuoka 421-0496 (JP)
(74) Representative: von Hirschhausen, Helge
(86) International application number: PCT/JP2016/082075
(87) International publication number: WO 2017/073731

(56) References cited:
- EP-A1- 2 361 643
- EP-A2- 1 655 044
- JP-A- 2013 248 334
- US-A1- 2013 303 963
- US-A1- 2015 297 816

## Description

### Technical Field

The present invention relates to a blood purification apparatus for purifying the blood of a patient while extracorporeally circulating the blood.

### Background Art

In general, a blood purification apparatus for giving dialysis treatment includes an arterial blood circuit and a venous blood circuit that form a blood circuit for extracorporeally circulating the blood of a patient, a blood purification device for purifying the blood that is extracorporeally circulated through the blood circuit, and an apparatus body provided with various treatment devices such as a blood pump for causing the blood circuit and the blood purification device to perform blood purification treatment. Vascular access catheters or puncture needles (an arterial puncture needle and a venous puncture needle) are attachable to the distal ends of the arterial blood circuit and the venous blood circuit, respectively.

For example, when the blood pump is activated after the arterial puncture needle and the venous puncture needle are stuck into the patient, the blood of the patient flows into the arterial blood circuit and the venous blood circuit. While the blood flows through the blood circuit, the blood is purified by the blood purification device. In the dialysis treatment, a dialysate introduction line for introducing dialysate into the blood purification device and a dialysate drain line for draining waste liquid from the blood purification device are connected to the blood purification device.

In the above blood purification apparatus, prior to the blood purification treatment, the blood circuit and the blood purification device need to be subjected to priming. Priming is a process in which a priming solution such as a physiological saline solution is supplied into the blood circuit by activating the blood pump, typically. In this process, a flow route in the blood circuit in which the blood extracorporeally circulates, a flow route in the blood purification device in which the blood flows (a blood flow route), and a flow route in the blood purification device in which the dialysate flows (a dialysate flow route) are filled with the priming solution. Such a technique has not been disclosed by any publicly known invention, and there is no information on patent literature to be cited.

### Summary of Invention

### Technical Problem

Typically, in the above known blood purification apparatus, after the filling with the priming solution, a washing process is required in which the flow routes are washed by further supplying the priming solution thereinto while draining the pre-supplied priming solution to the outside. Hence, in a known exemplary technique, a priming-solution storage bag storing the priming solution is connected to the distal end of the arterial blood circuit, while a collecting bag in which the priming solution is collected is connected to the distal end of the venous blood circuit. To perform the washing process, the blood pump is activated, whereby the priming solution is drained into the collecting bag. Such a known technique has a problem in that the collecting bag provided exclusively for collecting the priming solution during priming is necessary, which leads to a cost increase. Such examples are described in prior art documents EP 2361643 A1 and JP 2013248334 A.

The present invention has been conceived in view of the above circumstances and provides a blood purification apparatus that does not require a priming-solutioncollecting bag to be connected to the apparatus only when priming is performed, so that the cost for performing priming can be reduced.

### Solution to Problem

The invention is defined by the appended claims.

### Advantageous Effects of Invention

When the priming solution is drained during priming, the priming solution supplied into the blood circuit is allowed to be drained through the dialysate drain line. Therefore, no bag for collecting the priming solution that is connected only when priming is performed is necessary, and the cost for performing priming can be reduced.

According to the invention of Claim 2, the blood purification apparatus further includes the substitution line that allows the predetermined portion of the venous blood circuit and the dialysate introduction line to communicate with each other. Furthermore, when the priming solution is drained during priming, the priming solution supplied into the blood circuit is allowed to flow into the dialysate drain line through the substitution line and to be drained. Therefore, efficient washing with the use of the substitution line can be performed. Furthermore, substances that cannot pass through the blood purification membranes can be washed away.

According to the invention of Claim 3, the substitution line is provided with the substitution pump. Therefore, more efficient washing with the use of the substitution line can be performed.

According to the invention of Claim 4, when the priming solution is drained during priming, the apparatus is capable of alternately performing the blood-extraction-side draining step in which the priming solution in the arterial blood circuit is drained through the dialysate drain line and the blood-returning-side draining step in which the priming solution in the venous blood circuit is drained through the dialysate drain line. Therefore, a larger amount of priming solution can be made to flow into the flow route to be subjected to priming, and bubbles can be prevented from remaining therein.

According to the invention of Claim 1, the distal end of the arterial blood circuit is connectable to the arterial priming-solution storage bag that stores the priming solution while the distal end of the venous blood circuit is connectable to the venous priming-solution storage bag that stores the priming solution. Furthermore, when the priming solution is drained during priming, the priming solution in the arterial priming-solution storage bag or in the venous priming-solution storage bag is allowed to be supplied into the blood circuit. Therefore, the side of the blood circuit can be filled with the priming solution more assuredly and smoothly.

According to the invention of Claim 5, the distal ends of the arterial blood circuit and the venous blood circuit are connectable to each other. Furthermore, the priming-solution storage bag that stores the priming solution is connectable to the branch line branching off from the arterial blood circuit or from the venous blood circuit. Furthermore, when the priming solution is drained during priming, the priming solution in the priming-solution storage bag is allowed to be supplied into the blood circuit. Therefore, the side of the blood circuit can be filled with the priming solution more assuredly and smoothly. In addition, since only one priming-solution storage bag is to be prepared, the cost for performing priming can be reduced.

According to the invention of Claim 6, when the priming solution is drained during priming, the blood purification device is held with the blood delivery port oriented upward. Therefore, the blood flow route and the dialysate flow route of the blood purification device can be assuredly filled with the priming solution. In addition, the blood purification device does not need to be turned upside down by a medical staff at the beginning of blood purification treatment.

According to the invention of Claim 7, when the priming solution is supplied during priming, gas discharged into the venous blood circuit is collected in the blood-circuit-side air trap chamber, and the collected gas is allowed to be discharged to the outside through the air discharge line by activating the air-discharging device. Therefore, the gas substituted with the priming solution with the supply of the priming solution can be assuredly discharged to the outside with the use of the blood-circuit-side air trap chamber and the air-discharging device.

According to the invention of Claim 8, the blood purification apparatus further comprises the liquid-level-detecting device capable of detecting a liquid surface in the blood-circuit-side air trap chamber. Furthermore, the discharge of the gas by the air-discharging device is stopped on condition that a liquid surface at a predetermined level is detected by the liquid-level-detecting device. Therefore, the work of supplying the priming solution can be finished at the detection of the full substitution of the gas with the priming solution by the use of the liquid-level-detecting device. Hence, more efficient priming can be performed.

### Brief Description of Drawings

[Fig. 1] Fig. 1 is a schematic diagram illustrating a blood purification apparatus according to an embodiment of the present invention.
[Fig. 2] Fig. 2 is a schematic view illustrating a blood purification device applied to the blood purification apparatus.
[Fig. 3] Fig. 3 is a flow chart illustrating a process performed by the blood purification apparatus at the time of priming.
[Fig. 4] Fig. 4 is a schematic diagram illustrating the blood purification apparatus (in a priming-solution supplying step).
[Fig. 5] Fig. 5 is another schematic diagram illustrating the blood purification apparatus (in the priming-solution supplying step).
[Fig. 6] Fig. 6 is yet another schematic diagram illustrating the blood purification apparatus (in the priming-solution supplying step).
[Fig. 7] Fig. 7 is yet another schematic diagram illustrating the blood purification apparatus (in the priming-solution supplying step).
[Fig. 8] Fig. 8 is yet another schematic diagram illustrating the blood purification apparatus (in the priming-solution supplying step).
[Fig. 9] Fig. 9 is yet another schematic diagram illustrating the blood purification apparatus (in a washing step).
[Fig. 10] Fig. 10 is yet another schematic diagram illustrating the blood purification apparatus (in the washing step).
[Fig. 11] Fig. 11 is yet another schematic diagram illustrating the blood purification apparatus (in a bubble-purging step).
[Fig. 12] Fig. 12 is a schematic diagram illustrating a blood purification apparatus according to another embodiment of the present invention.
[Fig. 13] Fig. 13 is a schematic diagram illustrating an exemplary modification regarding the state of connection between a blood circuit and a priming-solution storage bag.
[Fig. 14] Fig. 14 is a schematic diagram illustrating another exemplary modification regarding the state of connection between the blood circuit and the priming-solution storage bag.

### Description of Embodiments

Embodiments of the present invention will now be described specifically with reference to the drawings.

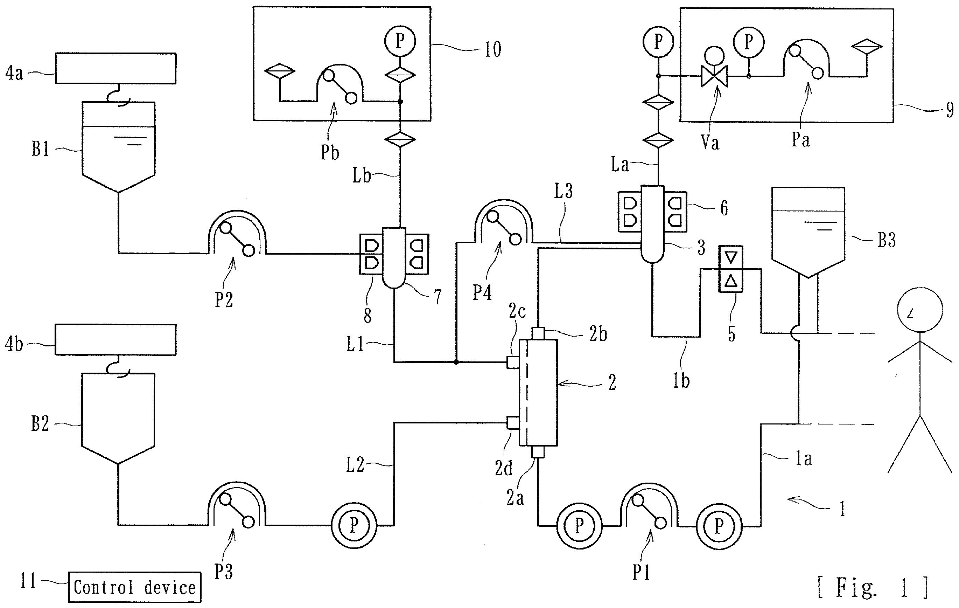

A blood purification apparatus according to an embodiment is applied to a hemodialysis apparatus for purifying the blood of a patient while extracorporeally circulating the blood. As illustrated in Fig. 1, the blood purification apparatus includes a blood circuit 1 including an arterial blood circuit 1a and a venous blood circuit 1b, a dialyzer 2 (a blood purification device) provided between the arterial blood circuit 1a and the venous blood circuit 1b and that purifies the blood flowing in the blood circuit 1, a blood pump P1 as a peristaltic pump that is provided to the arterial blood circuit 1a, a dialysate introduction line L1 and a dialysate drain line L2, a dialysate pump P2 and a waste-liquid pump P3 provided to the dialysate introduction line L1 and the dialysate drain line L2, respectively, a substitution line L3, a substitution pump P4 provided to the substitution line L3, and a control device 11. Reference character P in the drawing denotes a pressure-detecting device.

The arterial blood circuit 1a and the venous blood circuit 1b are each provided at the distal end thereof with a connector, to which an arterial puncture needle or a venous puncture needle (not illustrated) is connectable. When the blood pump P1 is activated (to undergo normal rotation) with the arterial puncture needle at the distal end of the arterial blood circuit 1a and the venous puncture needle at the distal end of the venous blood circuit 1b being stuck in the patient, the blood of the patient flows through the arterial blood circuit 1a into the dialyzer 2, where the blood is purified. The purified blood flows through the venous blood circuit 1b and returns into the body of the patient. Instead of sticking the arterial puncture needle and the venous puncture needle into blood vessels of the patient, a double-lumen catheter may be inserted into the subclavian vein or the femoral vein of the patient or into a blood vessel in an arm of the patient.

The venous blood circuit 1b is provided at a halfway position thereof with a blood-circuit-side air trap chamber 3. The blood that is extracorporeally circulated is subjected to bubble removal in the air trap chamber 3 and then returns into the patient. The venous blood circuit 1b is further provided at a distal portion thereof with a clamping device 5. When the clamping device 5 is opened or closed selectively, the flow route at that position can be opened or closed.

The venous blood circuit 1b according to the present embodiment includes the blood-circuit-side air trap chamber 3 provided at the connection to the substitution line L3, an air discharge line La extending from the top of the blood-circuit-side air trap chamber 3 and through which air in the blood-circuit-side air trap chamber 3 is allowed to be discharged, and an air-discharging device 9 capable of discharging air from the blood-circuit-side air trap chamber 3 through the air discharge line La to the outside. The blood-circuit-side air trap chamber 3 is provided with a liquid-level-detecting device 6 capable of detecting the level of the liquid surface in the blood-circuit-side air trap chamber 3.

The air-discharging device 9 includes a flow route communicating with the air discharge line La. The distal end of the flow route is open to the atmosphere through a filter. An electromagnetic valve Va and a peristaltic pump Pa are provided at respective halfway positions of the flow route. If the peristaltic pump Pa is activated to undergo normal rotation with the electromagnetic valve Va open, air in the blood-circuit-side air trap chamber 3 is released to the outside, whereby the liquid surface in the blood-circuit-side air trap chamber 3 can be raised. If the peristaltic pump Pa is activated to undergo reverse rotation, air is introduced into the blood-circuit-side air trap chamber 3, whereby the liquid surface in the blood-circuit-side air trap chamber 3 can be lowered.

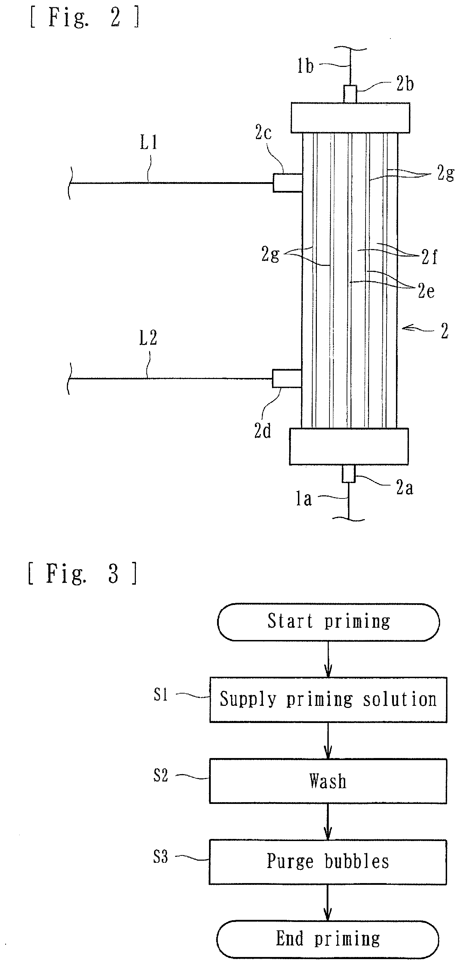

As illustrated in Fig. 2, the dialyzer 2 has a blood introduction port 2a that allows the blood to be introduced thereinto, a blood delivery port 2b that allows the blood to be delivered therefrom, blood flow routes 2e extending between the blood introduction port 2a and the blood delivery port 2b and through which the blood is allowed to flow, a dialysate introduction port 2c that allows dialysate to be introduced thereinto, a dialysate delivery port 2d that allows the dialysate to be delivered therefrom, dialysate flow route 2f extending between the dialysate introduction port 2c and the dialysate delivery port 2d and through which the dialysate is allowed to flow in a direction opposite to the direction in which the blood flows in the blood flow routes 2e, and blood purification membranes 2g interposed between the blood flow routes 2e and the dialysate flow route 2f and being capable of purifying the blood flowing in the blood flow routes 2e.

More specifically, the dialyzer 2 has a housing, from which the blood introduction port 2a, the blood delivery port 2b, the dialysate introduction port 2c, and the dialysate delivery port 2d project. The arterial blood circuit 1a is connected to the blood introduction port 2a. The venous blood circuit 1b is connected to the blood delivery port 2b. The dialysate introduction line L1 is connected to the dialysate introduction port 2c. The dialysate drain line L2 is connected to the dialysate delivery port 2d. For efficient dialysis treatment, the blood introduction port 2a serving as the inlet for the blood and the dialysate introduction port 2c serving as the inlet for the dialysate are positioned opposite each other in the top-bottom direction so that the dialysate flows in the direction opposite to the direction in which the blood in the blood flow routes 2e flows.

The dialyzer 2 is provided thereinside with a plurality of hollow fiber membranes. Such hollow fibers form the blood purification membranes 2g for purifying the blood. That is, the inside of the each of the blood purification membranes 2g forming the hollow fibers provides a corresponding one of the blood flow routes 2e, and the spaces between the housing and the hollow fibers form the dialysate flow route 2f. The blood purification membranes 2g as the hollow fiber membranes each have a number of very small holes (pores) each extending therethrough from the outer peripheral surface to the inner peripheral surface. Hence, impurities and the like contained in the blood flowing in the blood flow routes 2e can penetrate through (be filtered by) the membranes into the dialysate flowing in the dialysate flow route 2f.

The dialysate introduction line L1 provides a flow route for introducing the dialysate into the dialyzer 2. One end of the dialysate introduction line L1 is connected to the dialysate introduction port 2c of the dialyzer 2. The dialysate introduction line L1 is provided at a halfway position thereof with the dialysate pump P2. As with the blood pump P1, the dialysate pump P2 is a peristaltic pump that delivers liquid by squeezing a flexible tube forming the dialysate introduction line L1. The other end of the dialysate introduction line L1 is connected to a dialysate storage bag B1 that stores a predetermined amount of dialysate. When the dialysate pump P2 is activated (to undergo normal rotation), the dialysate in the dialysate storage bag B1 can be introduced into the dialyzer 2.

The dialysate drain line L2 provides a flow route for draining the waste liquid from the dialyzer 2. One end of the dialysate drain line L2 is connected to the dialysate delivery port 2d of the dialyzer 2. The dialysate drain line L2 is provided at a halfway position thereof with the waste-liquid pump P3. As with the blood pump P1, the waste-liquid pump P3 is a peristaltic pump that delivers liquid by squeezing a flexible tube forming the dialysate drain line L2. The other end of the dialysate drain line L2 is connected to a waste-liquid storage bag B2 that stores a predetermined amount of waste liquid. When the waste-liquid pump P3 is activated (to undergo normal rotation), the dialysate (the waste liquid) drained from the dialyzer 2 can be introduced into the waste-liquid storage bag B2.

Thus, the dialysate in the dialysate storage bag B1 flows toward and is supplied into the dialyzer 2 with the normal rotation of the dialysate pump P2, and the dialysate (waste liquid) in the dialyzer 2 flows toward and is drained into the waste-liquid storage bag B2 with the normal rotation of the waste-liquid pump P3. The dialysate storage bag B1 and the waste-liquid storage bag B2 are hung on hooks provided to weighing machines 4a and 4b, respectively. The weighing machines 4a and 4b are capable of measuring in real time the weights of the dialysate storage bag B1 and the waste-liquid storage bag B2, respectively. The dialysate storage bag B1 and the waste-liquid storage bag B2 are each a flexible storage case. Before the blood purification treatment is started, the waste-liquid storage bag B2 is empty with no waste liquid stored therein.

On the basis of the weights measured by the weighing machines 4a and 4b and preset values stored in advance, the driving of the blood pump P1, the dialysate pump P2, and the waste-liquid pump P3 (and the substitution pump P4) is controlled. If there is a treatment condition that ultrafiltration is not to be performed for the patient, the driving speed of the waste-liquid pump P3 is controlled to be the same as the driving speed of the dialysate pump P2. If ultrafiltration is to be performed, the driving speed of the waste-liquid pump P3 is controlled to be higher than the driving speed of the dialysate pump P2 by the flow rate for ultrafiltration. If there is any difference, after a predetermined period of time, between the weight of each of the dialysate storage bag B1 and the waste-liquid storage bag B2 that are detected by the weighing machines 4a and 4b and the theoretical weight, the driving speed of the waste-liquid pump P3 can be finely adjusted automatically so that correction for eliminating the difference is performed.

The substitution line L3 provides a flow route that allows a predetermined portion of the venous blood circuit 1b (in the present embodiment, the blood-circuit-side air trap chamber 3 provided to the venous blood circuit 1b) and the dialysate introduction line L1 to communicate with each other. The substitution line L3 is provided with the substitution pump P4. When the substitution pump P4 is activated (to undergo normal rotation), the dialysate in the dialysate introduction line L1 can be supplied into the venous blood circuit 1b for substitution (postsubstitution). As with the blood pump P1, the substitution pump P4 is a peristaltic pump that delivers liquid by squeezing a flexible tube forming the substitution line L3.

The dialysate introduction line L1 according to the present embodiment further includes a dialysate-side air trap chamber 7 provided between the dialysate pump P2 and the dialyzer 2, an air discharge line Lb extending from the top of the dialysate-side air trap chamber 7 and through which air in the dialysate-side air trap chamber 7 is allowed to be discharged, and an air-discharging device 10 capable of discharging air from the dialysate-side air trap chamber 7 through the air discharge line Lb to the outside. The dialysate-side air trap chamber 7 is provided with a liquid-level-detecting device 8 capable of detecting the level of the liquid surface in the dialysate-side air trap chamber 7.

The air-discharging device 10 includes a flow route communicating with the air discharge line Lb. The distal end of the flow route is open to the atmosphere through a filter. A peristaltic pump Pb is provided at a halfway position of the flow route. If the peristaltic pump Pb is activated to undergo normal rotation, air in the dialysate-side air trap chamber 7 is released to the outside, whereby the liquid surface in the dialysate-side air trap chamber 7 can be raised. If the peristaltic pump Pb is activated to undergo reverse rotation, air is introduced into the dialysate-side air trap chamber 7, whereby the liquid surface in the dialysate-side air trap chamber 7 can be lowered.

The control device 11 is a microcomputer or the like included in the blood purification apparatus. The control device 11 is electrically connected to each of the actuators (the blood pump P1, the dialysate pump P2, the waste-liquid pump P3, the substitution pump P4, and the like), the sensors (the liquid-level-detecting devices 6 and 8 and the like), and the valve devices (the clamping device 5 and the like) and controls such devices in various ways. In the present embodiment, with such control operations performed by the control device 11, priming prior to the blood purification treatment can be performed automatically.

Priming is a process that is performed by supplying a priming solution into the blood circuit 1 (the arterial blood circuit 1a and the venous blood circuit 1b) with the activation of the blood pump P1. As illustrated in Fig. 3, the priming process includes a priming-solution-supplying step S1, a washing step S2, and a bubble-purging step S3. In the priming-solution-supplying step S1, the flow routes in the blood circuit 1 and the blood flow routes 2e and the dialysate flow route 2f in the dialyzer 2 are filled with the priming solution, whereby the gas (air) therein is substituted with the priming solution. In the washing step S2, washing is performed by supplying fresh priming solution while draining the priming solution supplied in the priming-solution-supplying step S1 to the outside. In the bubble-purging step S3, the priming solution is made to circulate through the flow routes that have been filled and washed with the priming solution, whereby bubbles remaining therein are collected and discharged to the outside.

The distal ends of the arterial blood circuit la and the venous blood circuit 1b according to the present embodiment are connectable to a priming-solution storage bag B3 that stores the priming solution (a physiological saline solution). Hence, at the time of supplying the priming solution in priming, the priming solution in the priming-solution storage bag B3 can be supplied into the blood circuit 1. In this present embodiment, the priming-solution storage bag B3 is connected to both of the distal ends of the arterial blood circuit 1a and the venous blood circuit 1b. Note that the priming-solution storage bag B3 only needs to be connected to at least the distal end of the arterial blood circuit 1a.

As illustrated in Fig. 1, the distal ends of the blood circuits 1a and 1b be both connectable to one priming-solution storage bag B3 that stores the priming solution and is shared by the arterial blood circuit la and the venous blood circuit 1b so that the priming solution in the priming-solution storage bag B3 can be supplied into the blood circuit 1 when the priming solution is drained during priming.

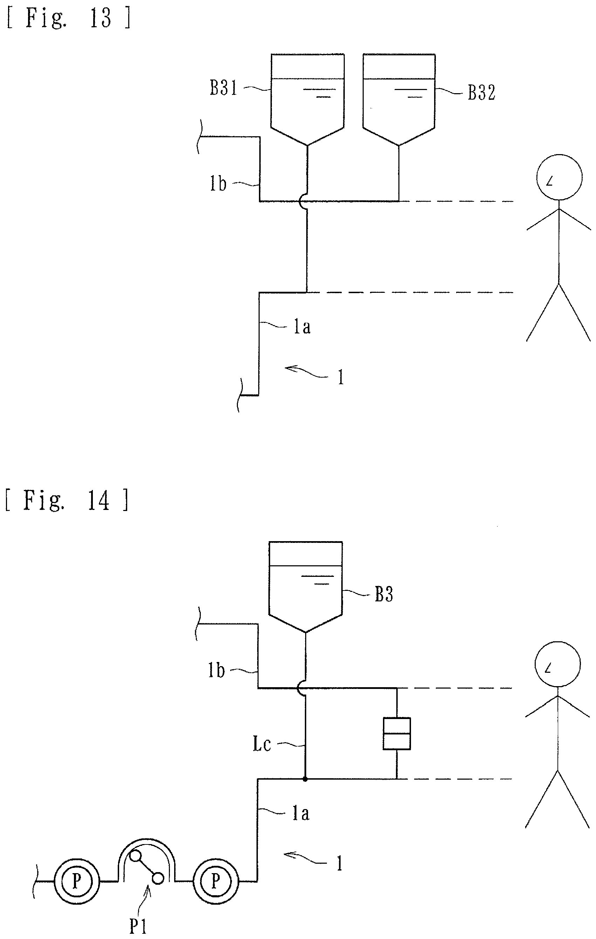

As illustrated in Fig. 13 an arterial priming-solution storage bag B31 that stores the priming solution is connectable to the distal end of the arterial blood circuit 1a while a venous priming-solution storage bag B32 that stores the priming solution is connectable to the distal end of the venous blood circuit 1b so that the priming solution in the arterial priming-solution storage bag B31 or in the venous priming-solution storage bag B32 can be supplied into the blood circuit 1 when the priming solution is drained during priming.

A configuration illustrated in Fig. 14 is also acceptable in which the distal ends of the arterial blood circuit 1a and the venous blood circuit 1b are connectable to each other, and the priming-solution storage bag B3 that stores the priming solution is connectable to a branch line Lc branching off from the arterial blood circuit 1a (or from the venous blood circuit 1b) so that the priming solution in the priming-solution storage bag B3 can be supplied into the blood circuit 1 when the priming solution is drained during priming. The branch line Lc preferably branches off from a position between the distal end of the arterial blood circuit 1a and the blood pump P1 or from any position of the venous blood circuit 1b.

In the blood purification apparatus according to the present embodiment, priming can be performed as follows. While the priming solution is supplied during priming (in the priming-solution-supplying step S1), the dialyzer 2 (the blood purification device) is held with the blood delivery port 2b oriented upward (with the dialysate introduction port 2c positioned above the dialysate delivery port 2d) as illustrated in Fig. 2. Furthermore, the priming solution introduced from the blood introduction port 2a into the blood flow routes 2e of the dialyzer 2 is filtered through the blood purification membranes 2g into the dialysate flow route 2f and is delivered from the dialysate introduction port 2c, whereby the dialysate flow route 2f are subjected to priming.

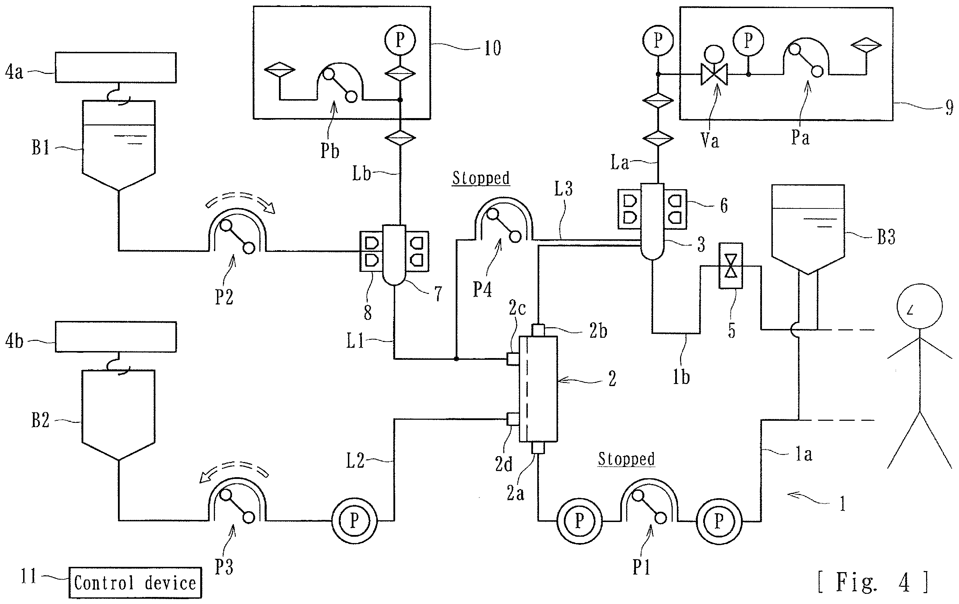

For example, when the priming solution starts to be supplied in priming (in the priming-solution-supplying step S1), as illustrated in Fig. 4, the dialysate pump P2 and the waste-liquid pump P3 are activated to undergo normal rotation at the same flow rate, with the clamping device 5 closed. Meanwhile, the other pumps such as the blood pump P1 and the substitution pump P4 are kept stopped. Thus, the dialysate in the dialysate storage bag B1 can be made to flow up to the connection between the dialysate introduction line L1 and the substitution line L3 and that part can be filled with the priming solution.

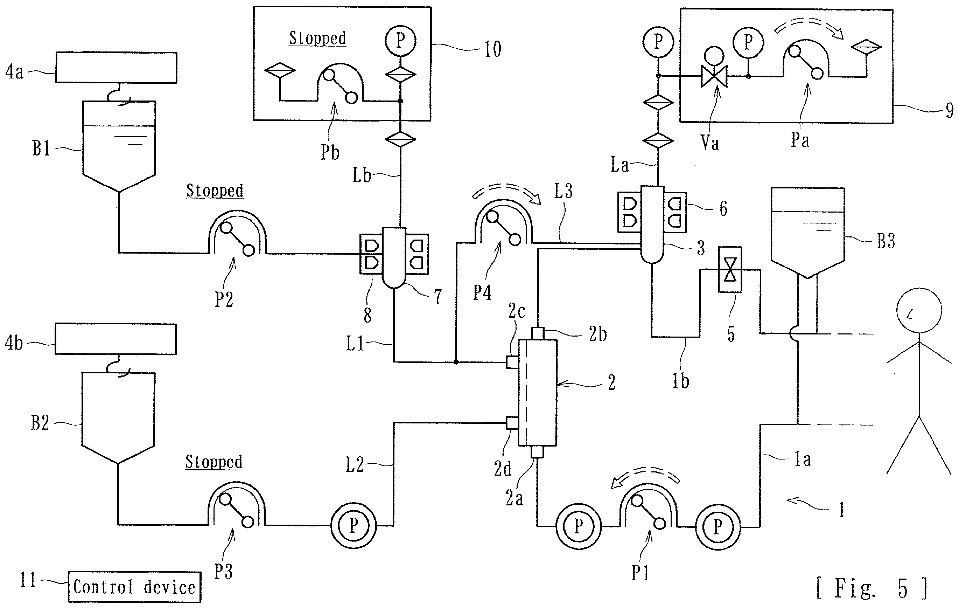

Then, the dialysate pump P2 and the waste-liquid pump P3 are stopped. Subsequently, as illustrated in Fig. 5, the peristaltic pump Pa is activated to undergo normal rotation with the clamping device 5 closed and the electromagnetic valve Va of the air-discharging device 9 open, and the blood pump P1 and the substitution pump P4 are activated to undergo normal rotation at the same flow rate. Thus, air in the arterial blood circuit 1a, in the dialysate flow route 2f of the dialyzer 2, and in the flow route provided by the substitution line L3 can be substituted with the priming solution. Then, at the detection of a liquid surface at a predetermined level by the liquid-level-detecting device 6, the blood pump P1 and the substitution pump P4 are stopped, the electromagnetic valve Va of the air-discharging device 9 is closed, and the peristaltic pump Pa is stopped. That is, the discharge of the gas by the air-discharging device 9 is stopped on condition that a liquid surface at a predetermined level is detected by the liquid-level-detecting device 6.

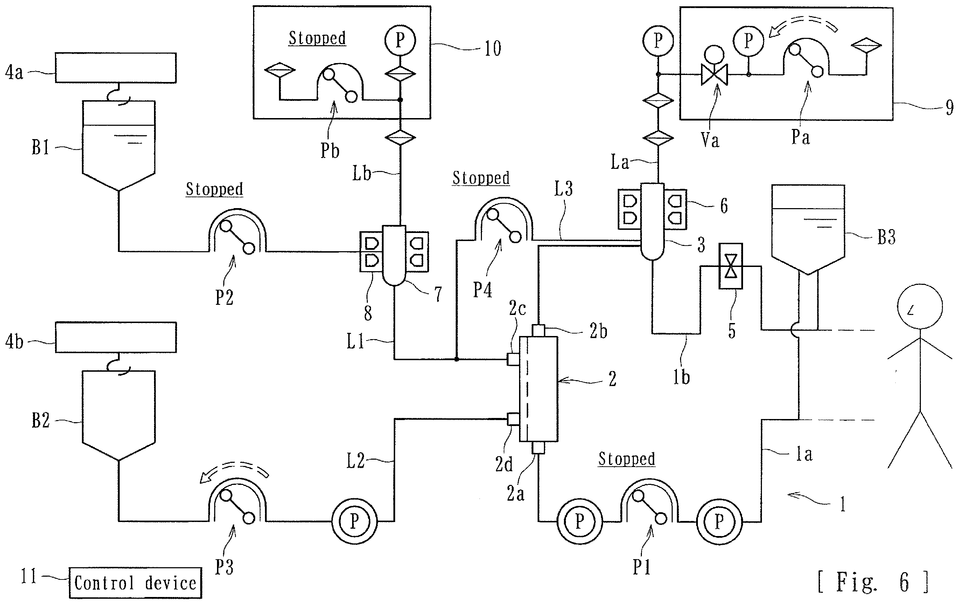

After the above operation is complete, as illustrated in Fig. 6, the waste-liquid pump P3 is activated to undergo normal rotation, and the peristaltic pump Pa is activated to undergo reverse rotation with the electromagnetic valve Va of the air-discharging device 9 open, whereby air is taken into the blood-circuit-side air trap chamber 3. In this step, the clamping device 5 is kept closed, and the pumps excluding the waste-liquid pump P3 and the peristaltic pump Pa of the air-discharging device 9 are kept stopped. Then, at the detection of a liquid surface at a predetermined level by the liquid-level-detecting device 6, the waste-liquid pump P3 and the peristaltic pump Pa of the air-discharging device 9 are stopped.

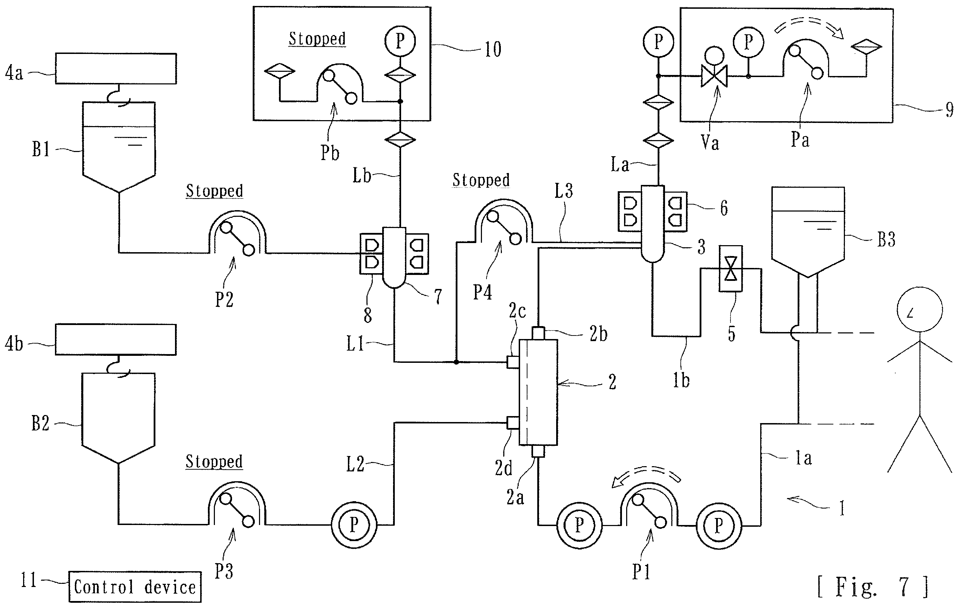

Subsequently, as illustrated in Fig. 7, the blood pump P1 is activated to undergo normal rotation at a predetermined flow rate, and the peristaltic pump Pa is activated to undergo normal rotation with the electromagnetic valve Va of the air-discharging device 9 open. In this step, the clamping device 5 is kept closed, and the pumps excluding the blood pump P1 and the peristaltic pump Pa of the air-discharging device 9 are kept stopped. Then, at the detection of a liquid surface at a predetermined level by the liquid-level-detecting device 6, the blood pump P1 and the peristaltic pump Pa of the air-discharging device 9 are stopped. Thus, air in the blood flow routes 2e of the dialyzer 2 and in the flow route provided by the venous blood circuit 1b from the connection to the dialyzer 2 up to the blood-circuit-side air trap chamber 3 can be substituted with the priming solution.

Furthermore, as illustrated in Fig. 6, the waste-liquid pump P3 is activated to undergo normal rotation, and the peristaltic pump Pa is activated to undergo reverse rotation with the electromagnetic valve Va of the air-discharging device 9 open, whereby air is taken into the blood-circuit-side air trap chamber 3. In this step, the clamping device 5 is kept closed, and the pumps excluding the waste-liquid pump P3 and the peristaltic pump Pa of the air-discharging device 9 are kept stopped. Then, at the detection of a liquid surface at a predetermined level by the liquid-level-detecting device 6, the waste-liquid pump P3 and the peristaltic pump Pa of the air-discharging device 9 are stopped.

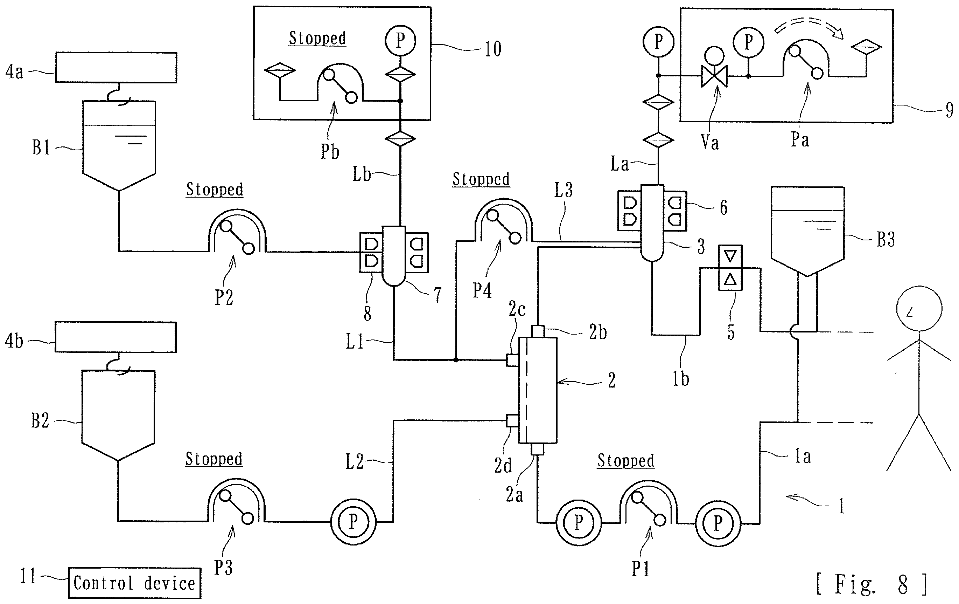

Subsequently, as illustrated in Fig. 8, the peristaltic pump Pa is activated to undergo normal rotation with the clamping device 5 open and the electromagnetic valve Va of the air-discharging device 9 open. Then, at the detection of a liquid surface at a predetermined level by the liquid-level-detecting device 6, the peristaltic pump Pa of the air-discharging device 9 is stopped. Thus, air in the flow route from the distal end of the venous blood circuit 1b (in the present embodiment, the portion connected to the priming-solution storage bag B3) up to the connection to the blood-circuit-side air trap chamber 3 can be substituted with the priming solution.

This is the end of the priming-solution-supplying step S1, and the process proceeds to the washing step S2. In the washing step S2 according to the present embodiment, the priming solution supplied into the blood circuit 1 can be drained through the dialysate drain line L2. More specifically, in the present embodiment, when the priming solution is drained during priming (in the washing step S2), the priming solution supplied into the blood circuit 1 is made to flow through the substitution line L3 into the dialysate drain line L2, whereby the priming solution can be drained.

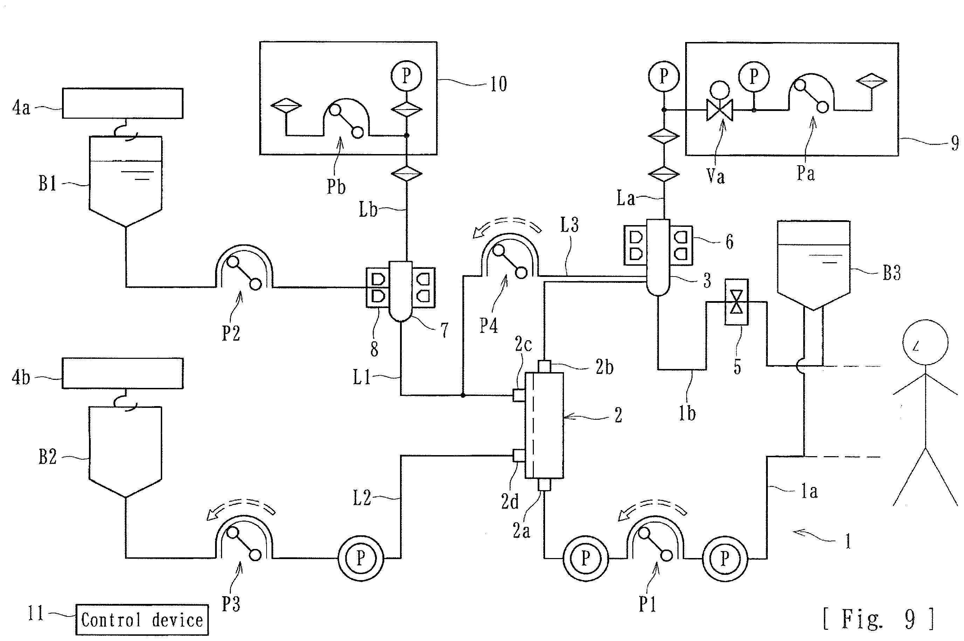

For example, as illustrated in Fig. 9, with the clamping device 5 closed, the blood pump P1 and the waste-liquid pump P3 are activated to undergo normal rotation while the substitution pump P4 is activated to undergo reverse rotation. In this step, the flow rate of the waste-liquid pump P3 and the flow rate of the blood pump P1 are made the same. With the normal rotation of the blood pump P1 and the waste-liquid pump P3 and the reverse rotation of the substitution pump P4, the priming solution is made to flow at a predetermined flow rate. Then, the blood pump P1, the waste-liquid pump P3, and the substitution pump P4 are stopped. Thus, a blood-extraction-side draining step in which the priming solution on the side of the arterial blood circuit la is drained through the dialysate drain line L2 can be performed.

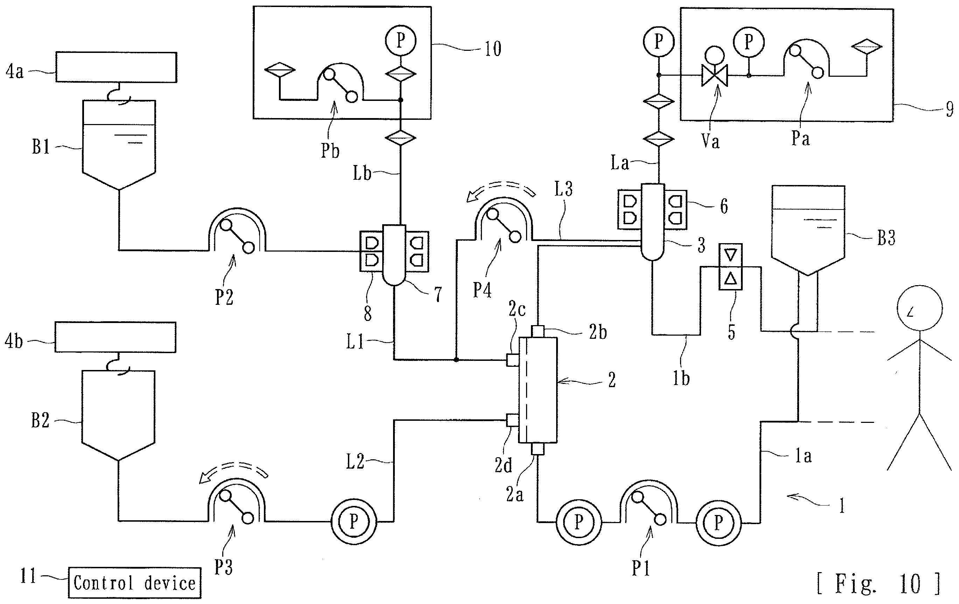

Subsequently, as illustrated in Fig. 10, with the clamping device 5 open, the waste-liquid pump P3 is activated to undergo normal rotation while the substitution pump P4 is activated to undergo reverse rotation. In this step, the flow rate of the waste-liquid pump P3 and the flow rate of the substitution pump P4 are made the same. With the normal rotation of the waste-liquid pump P3 and the reverse rotation of the substitution pump P4, the priming solution is made to flow at a predetermined flow rate. Then, the waste-liquid pump P3 and the substitution pump P4 are stopped. Thus, a blood-returning-side draining step in which the priming solution on the side of the venous blood circuit 1b is drained through the dialysate drain line L2 can be performed. Accordingly, the blood-extraction-side draining step (see Fig. 9) and the blood-returning-side draining step (see Fig. 10) can be performed alternately. By repeating these two steps a plurality of times, a larger amount of priming solution can be made to flow.

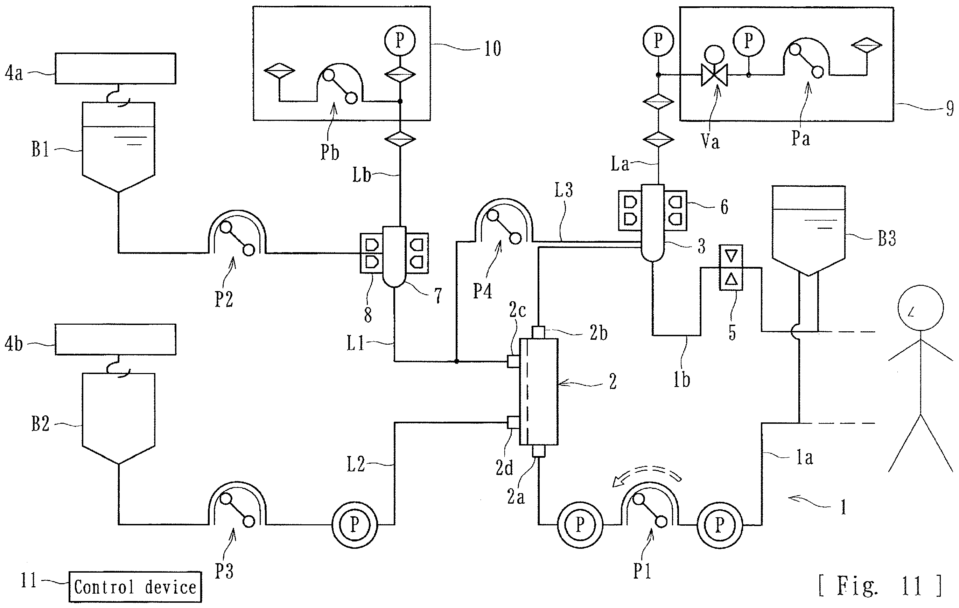

This is the end of the washing step S2, and the process proceeds to the bubble-purging step S3. In the bubble-purging step S3, as illustrated in Fig. 11, with the clamping device 5 open, the blood pump P1 is activated to undergo normal rotation. In this step, the pumps excluding the blood pump P1 are kept stopped. Thus, the priming solution circulates through the arterial blood circuit 1a and the venous blood circuit 1b, and bubbles generated in the circulation are collected in the blood-circuit-side air trap chamber 3.

If a liquid surface at a predetermined level is no longer detected by the liquid-level-detecting device 6, the peristaltic pump Pa of the air-discharging device 9 is driven until a liquid surface at the predetermined level is detected by the liquid-level-detecting device 6. Then, at the detection of the liquid surface at the predetermined level by the liquid-level-detecting device 6, the blood pump P1 and the peristaltic pump Pa of the air-discharging device 9 are stopped. Thus, the bubble-purging step S3 ends, and the priming process ends. If blood purification treatment is performed after priming is finished, the priming-solution storage bag B3 is removed from the distal ends of the arterial blood circuit 1a and the venous blood circuit 1b, and the respective puncture needles are connected thereto.

According to the above embodiment, when the priming solution is drained during priming (in the washing step S2), the priming solution supplied into the blood circuit 1 can be drained through the dialysate drain line L2. Therefore, no bag for collecting the priming solution that is connected only when priming is performed is necessary, and the cost for performing priming can be reduced. Specifically, the present embodiment employs the substitution line L3 that allows a predetermined portion of the venous blood circuit 1b and the dialysate introduction line L1 to communicate with each other. Furthermore, when the priming solution is drained during priming, the priming solution supplied into the blood circuit 1 can be made to flow through the substitution line L3 up to the dialysate drain line L2 and can be drained therefrom. Therefore, efficient washing with the use of the substitution line L3 can be performed. Furthermore, substances that cannot pass through the blood purification membranes can be washed away.

Furthermore, when the priming solution is drained during priming, the blood-extraction-side draining step in which the priming solution on the side of the arterial blood circuit 1a is drained through the dialysate drain line L2 and the blood-returning-side draining step in which the priming solution on the side of the venous blood circuit 1b is drained through the dialysate drain line L2 are performed alternately. Therefore, a larger amount of priming solution can be made to flow into the flow route to be subjected to priming, and bubbles can be prevented from remaining therein.

Furthermore, regarding the arterial blood circuit la and the venous blood circuit 1b, at least the distal end of the arterial blood circuit 1a is connectable to the priming-solution storage bag B3 that stores the priming solution, and the priming solution in the priming-solution storage bag B3 can be supplied into the blood circuit 1 when the priming solution is drained during priming. Therefore, the side of the blood circuit 1 can be filled with the priming solution more assuredly and smoothly.

Furthermore, when the priming solution is drained during priming, the dialyzer 2 is held with the blood delivery port 2b oriented upward. Therefore, the blood flow routes 2e and the dialysate flow route 2f of the dialyzer 2 can be assuredly filled with the priming solution. In addition, the dialyzer 2 does not need to be turned upside down by a medical staff at the beginning of blood purification treatment. In this case, it is preferable that, when the priming solution is supplied during priming, the dialyzer 2 be held with the blood delivery port 2b oriented upward, and the priming solution (the physiological saline solution in the priming-solution storage bag B3) introduced from the blood introduction port 2a into the blood flow routes 2e of the dialyzer 2 be filtered through the blood purification membranes 2g into the dialysate flow route 2f and be delivered from the dialysate introduction port 2c so that the dialysate flow route 2f can be subjected to priming. Thus, the blood flow routes 2e and the dialysate flow route 2f of the dialyzer 2 can be assuredly filled and washed with the priming solution. In addition, the dialyzer 2 does not need to be turned upside down by a medical staff at the beginning of blood purification treatment. Alternatively, the dialyzer 2 may be held with the blood delivery port 2b oriented downward while priming of the dialysate flow route 2f is performed, and the dialyzer 2 may be turned upside down at the beginning of blood purification treatment.

Furthermore, when the priming solution is supplied during priming, the gas discharged into the venous blood circuit 1b can be collected in the blood-circuit-side air trap chamber 3 and then be discharged to the outside through the air discharge line La by activating the air-discharging device 9. Therefore, when the gas substituted with the priming solution with the supply of the priming solution can be assuredly discharged to the outside with the use of the blood-circuit-side air trap chamber 3 and the air-discharging device 9.

Furthermore, the liquid-level-detecting device 6 capable of detecting the liquid surface in the blood-circuit-side air trap chamber 3 is employed, and the discharge of the gas by the air-discharging device 9 is stopped on condition that a liquid surface at a predetermined level is detected by the liquid-level-detecting device 6. Therefore, the work of supplying the priming solution can be finished at the detection of the full substitution of the gas with the priming solution by the use of the liquid-level-detecting device 6. Hence, more efficient priming can be performed.

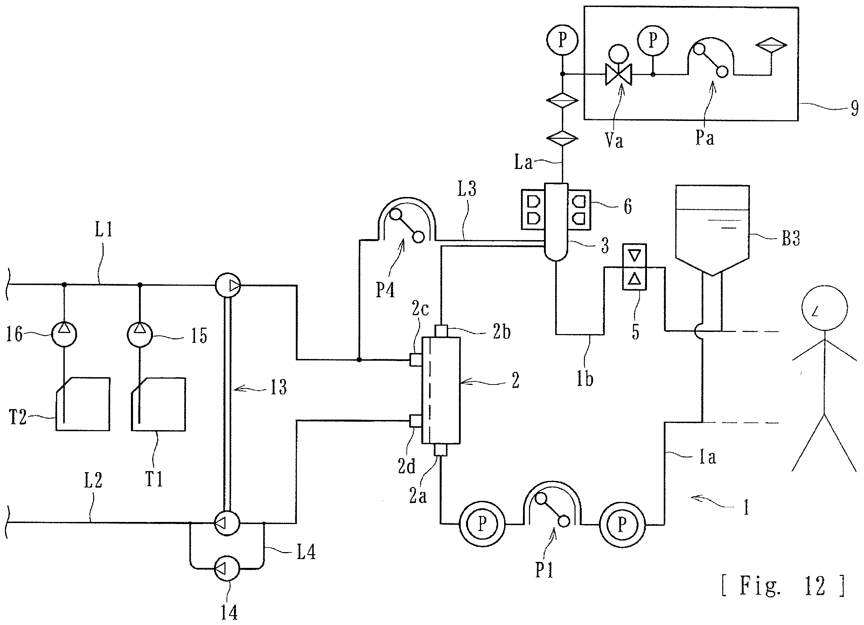

While an embodiment has been described above, the present invention is not limited thereto. For example, as illustrated in Fig. 12, the present invention may be applied to a blood purification apparatus including a duplex pump 13 provided over the dialysate introduction line L1 and the dialysate drain line L2, a bypass line L4 connected to the dialysate drain line L2 and that bypasses the duplex pump 13, and an ultrafiltration pump 14 connected to the bypass line L4. The dialysate introduction line L1 of such a blood purification apparatus is connected to a water-supplying device (not illustrated), to a storage tank T1 that stores an A undiluted solution, and to a storage tank T2 that stores a B undiluted solution. Pumps 15 and 16 are activated to supply the A undiluted solution and the B undiluted solution into the dialysate introduction line L1, whereby a dialysate at a predetermined concentration is prepared and is introduced into the dialyzer 2. In such a case also, when the priming solution is drained during priming, the priming solution supplied into the blood circuit 1 can be drained through the dialysate drain line L2.

In the above embodiment, when the priming solution is drained during priming, the priming solution supplied into the blood circuit 1 can be made to flow into the dialysate drain line L2 through the substitution line L3 and be drained therefrom. Alternatively, after the priming solution is introduced into the blood flow routes 2e of the dialyzer 2, the priming solution may be filtered through the blood purification membranes 2g into the dialysate flow route 2f, be made to flow into the dialysate drain line L2, and be drained therefrom. Moreover, the above embodiment may be applied to a blood purification apparatus that includes none of the blood-circuit-side air trap chamber 3, the dialysate-side air trap chamber 7, and the air-discharging devices 9 and 10, or to a blood purification apparatus that does not include the substitution line L3 and the substitution pump P4. In addition, the blood purification device is not limited to a device including the blood purification membranes 2g formed of hollow fiber membranes and may be any other device that is capable of giving treatment by purifying blood. The applicable blood purification treatment is not limited to dialysis treatment and may be any other treatment in which the blood of a patient is purified while being extracorporeally circulated.

### Industrial Applicability

The blood purification apparatus may have any other additional functions or the like, as long as the apparatus is configured such that, when the priming solution is drained during priming, a priming solution supplied into a blood circuit can be drained through a dialysate drain line. Reference Signs List

- 1: blood circuit
- la: arterial blood circuit
- 1b: venous blood circuit
- 2: dialyzer (blood purification device)
- 3: blood-circuit-side air trap chamber
- 4a, 4b: weighing machine
- 5: clamping device
- 6: liquid-level-detecting device
- 7: dialysate-side air trap chamber
- 8: liquid-level-detecting device
- 9, 10: air-discharging device
- 11: control device
- L1: dialysate introduction line
- L2: dialysate drain line
- L3: substitution line
- P1: blood pump
- P2: dialysate pump
- P3: waste-liquid pump
- P4: substitution pump

## Claims

1. A blood purification apparatus comprising
a blood purification device (2) including blood purification membranes (2g) that are capable of purifying blood;
a blood circuit (1) including an arterial blood circuit (1a) and a venous blood circuit (1b) that are connected to the blood purification device (2), the blood circuit (1) allowing blood of a patient to extracorporeally circulate from a distal end of the arterial blood circuit (1a) to a distal end of the venous blood circuit (1b);
a dialysate introduction line (L1) connected to the blood purification device (2) and that allows dialysate to be introduced into the blood purification device (2),
a dialysate drain line (L2) connected to the blood purification device (2) and that allows waste liquid to be drained from the blood purification device (2); and
a blood pump (P1) provided to the arterial blood circuit (1a) and that is capable of causing the blood of the patient to extracorporeally circulate from the arterial blood circuit (1a) toward the venous blood circuit (1b),
the blood purification apparatus being capable of performing priming by supplying a priming solution into the blood circuit (1) with an activation of the blood pump (P1),
wherein, when the priming solution is drained during priming, the priming solution supplied into the blood circuit (1) is allowed to be drained through the dialysate drain line (L2),
**characterized in that**
(i) the distal ends of the arterial blood circuit (1a) and the venous blood circuit (1b) are both connectable to one priming-solution storage bag (B3) that stores the priming solution and to be shared by the two blood circuits (1a, 1b), and **in that**, when the priming solution is drained during priming, the priming solution in the priming-solution storage bag (B3) is allowed to be supplied into the blood circuit (1), and/or
(ii) the distal end of the arterial blood circuit (1a) is connectable to an arterial priming-solution storage bag (B31) that stores the priming solution while the distal end of the venous blood circuit (1b) is connectable to a venous priming-solution storage bag (B32) being comprised by the blood purification apparatus and that is configured to store the priming solution, and **in that**, when the priming solution is drained during priming, the priming solution in the arterial priming-solution storage bag (B31) or in the venous priming-solution storage bag (B32) is allowed to be supplied into the blood circuit (1).

2. The blood purification apparatus according to Claim 1, **characterized by** further including a substitution line (L3) that allows a predetermined portion of the venous blood circuit (1b) and the dialysate introduction line (L1) to communicate with each other, the apparatus being further **characterized in that**, when the priming solution is drained during priming, the priming solution supplied into the blood circuit (1) is allowed to flow into the dialysate drain line (L2) through the substitution line (L3) and to be drained.

3. The blood purification apparatus according to Claim 2, **characterized in that** the substitution line (L3) is provided with a substitution pump (P4).

4. The blood purification apparatus according to any of Claims 1 to 3, **characterized in that**, when the priming solution is drained during priming, the apparatus is capable of alternately performing a blood-extraction-side draining step in which the priming solution in the arterial blood circuit (1a) is drained through the dialysate drain line (L2) and a blood-returning-side draining step in which the priming solution in the venous blood circuit (1b) is drained through the dialysate drain line (L2).

5. The blood purification apparatus according to any of Claims 1 to 4, **characterized in that** the distal ends of the arterial blood circuit (1a) and the venous blood circuit (1b) are connectable to each other, **in that** a priming-solution storage bag (B3) that stores the priming solution is connectable to a branch line branching off from the arterial blood circuit (1a) or from the venous blood circuit (1b),and **in that**, when the priming solution is drained during priming, the priming solution in the priming-solution storage bag (B3) is allowed to be supplied into the blood circuit (1).

6. The blood purification apparatus according to any of Claims 1 to 5, **characterized in that** the blood purification device (2) has a blood introduction port (2a) that allows the blood to be introduced into the blood purification device (2), a blood delivery port (2b) that allows the blood to be delivered from the blood purification device (2), a blood flow route (2e) extending between the blood introduction port (2a) and the blood delivery port (2b) and through which the blood is allowed to flow, a dialysate introduction port (2c) that allows dialysate to be introduced into the blood purification device (2), a dialysate delivery port (2d) that allows the dialysate to be delivered from the blood purification device (2), and a dialysate flow route (2f) extending between the dialysate introduction port (2c) and the dialysate delivery port (2d) and through which the dialysate is allowed to flow in a direction opposite to a direction in which the blood flows in the blood flow route (2e), the apparatus being further **characterized in that**, when the priming solution is drained during priming, the blood purification device (2) is held with the blood delivery port (2b) oriented upward.

7. The blood purification apparatus according to any of Claims 2 to 6, **characterized by** further comprising:
a blood-circuit-side air trap chamber (3) provided at a connection between the venous blood circuit (1b) and the substitution line (L3);
an air discharge line (La) extending from a top of the blood-circuit-side air trap chamber (3) and through which air in the blood-circuit-side air trap chamber (3) is allowed to be discharged; and
an air-discharging device (9) capable of discharging the air in the blood-circuit-side air trap chamber (3) to an outside through the air discharge line,
the apparatus being **characterized in that**, when the priming solution is supplied during priming, gas discharged into the venous blood circuit (1b) is collected in the blood-circuit-side air trap chamber (3), and the collected gas is allowed to be discharged to the outside through the air discharge line (La) by activating the air-discharging device (9).

8. The blood purification apparatus according to Claim 7, **characterized by** further comprising a liquid-level-detecting device (6) capable of detecting a liquid surface in the blood-circuit-side air trap chamber (3), the apparatus being further **characterized in that** the discharge of the gas by the air-discharging device (9) is stopped on condition that a liquid surface at a predetermined level is detected by the liquid-level-detecting device (6).

## Patentansprüche

1. Ein Blutreinigungsapparat, umfassend
eine Blutreinigungsvorrichtung (2), die Blutreinigungsmembranen (2g) aufweist, die in der Lage sind, Blut zu reinigen;
einen Blutkreislauf (1), der einen arteriellen Blutkreislauf (1a) und einen venösen Blutkreislauf (1b) aufweist, die mit der Blutreinigungsvorrichtung (2) verbunden sind, wobei der Blutkreislauf (1) es dem Blut eines Patienten ermöglicht, extrakorporal von einem distalen Ende des arteriellen Blutkreislaufs (1a) zu einem distalen Ende des venösen Blutkreislaufs (1b) zu zirkulieren;
eine Dialysateinführungsleitung (L1), die mit der Blutreinigungsvorrichtung (2) verbunden ist und die es ermöglicht, Dialysat in die Blutreinigungsvorrichtung (2) einzuführen,
eine Dialysatablassleitung (L2), die mit der Blutreinigungsvorrichtung (2) verbunden ist und das Ablassen von Abfallflüssigkeit aus der Blutreinigungsvorrichtung (2) ermöglicht; und
eine Blutpumpe (P1), die an den arteriellen Blutkreislauf (1a) angeschlossen und in der Lage ist, das Blut des Patienten extrakorporal vom arteriellen Blutkreislauf (1a) zum venösen Blutkreislauf (1b) zirkulieren zu lassen,
wobei der Blutreinigungsapparat in der Lage ist, ein Priming durchzuführen, indem eine Priming-Lösung in den Blutkreislauf (1) mit einer Aktivierung der Blutpumpe (P1) zugeführt wird, wobei, wenn die Priming-Lösung während des Primings abgelassen wird, die in den Blutkreislauf (1) zugeführte Priming-Lösung durch die Dialysatablassleitung (L2) abgelassen werden kann,
**dadurch gekennzeichnet, dass**
(i) die distalen Enden des arteriellen Blutkreislaufs (1a) und des venösen Blutkreislaufs (1b) beide mit einem Vorratsbeutel (B3) für die Priming-Lösung verbunden werden können, der die Priming-Lösung speichert und von den beiden Blutkreisläufen (1a, 1b) gemeinsam genutzt wird, und dass, wenn die Priming-Lösung während des Primings abgelassen wird, die Priming-Lösung in dem Vorratsbeutel (B3) für die Priming-Lösung in den Blutkreislauf (1) zugeführt werden kann, und/oder
(ii) das distale Ende des arteriellen Blutkreislaufs (1a) mit einem arteriellen Vorratsbeutel (B31) für die Priming-Lösung verbindbar ist, der die Priming-Lösung speichert, während das distale Ende des venösen Blutkreislaufs (1b) mit einem venösen Vorratsbeutel (B32) für die Priming-Lösung verbindbar ist, der von dem Blutreinigungsapparat umfasst ist und der ausgebildet ist, um die Priming-Lösung zu speichern, und dass, wenn die Priming-Lösung während des Primings abgelassen wird, die Priming-Lösung in dem arteriellen Vorratsbeutel (B31) für die Priming-Lösung oder in dem venösen Vorratsbeutel (B32) für die Priming-Lösung in den Blutkreislauf (1) zugeführt werden kann.

2. Blutreinigungsapparat nach Anspruch 1, **dadurch gekennzeichnet, dass** er ferner eine Substitutionsleitung (L3) aufweist, die es ermöglicht, dass ein vorbestimmter Abschnitt des venösen Blutkreislaufs (1b) und die Dialysateinführungsleitung (L1) miteinander in Verbindung stehen, wobei der Apparat ferner **dadurch gekennzeichnet ist, dass**, wenn die Priming-Lösung während des Primings abgelassen wird, die in den Blutkreislauf (1) zugeführte Priming-Lösung durch die Substitutionsleitung (L3) in die Dialysatablassleitung (L2) fließen und abgelassen werden kann.

3. Blutreinigungsapparat nach Anspruch 2, **dadurch gekennzeichnet, dass** die Substitutionsleitung (L3) mit einer Substitutionspumpe (P4) versehen ist.

4. Blutreinigungsapparat nach einem der Ansprüche 1 bis 3, **dadurch gekennzeichnet, dass** der Apparat in der Lage ist, beim Ablassen der Priming-Lösung während des Primings abwechselnd einen blutentnahmeseitigen Ablassschritt, bei dem die Priming-Lösung im arteriellen Blutkreislauf (1a) über die Dialysatablassleitung (L2) abgelassen wird, und einen blutrückführseitigen Ablassschritt, bei dem die Priming-Lösung im venösen Blutkreislauf (1b) über die Dialysatablassleitung (L2) abgelassen wird, durchzuführen.

5. Blutreinigungsapparat nach einem der Ansprüche 1 bis 4, **dadurch gekennzeichnet, dass** die distalen Enden des arteriellen Blutkreislaufs (1a) und des venösen Blutkreislaufs (1b) miteinander verbindbar sind, dass ein die Priming-Lösung speichernder Vorratsbeutel (B3) mit einer vom arteriellen Blutkreislauf (1a) oder vom venösen Blutkreislauf (1b) abzweigenden Zweigleitung verbindbar ist, und dass beim Ablassen der Priming-Lösung während des Primings die Priming-Lösung im Vorratsbeutel (B3) in den Blutkreislauf (1) einspeisbar ist.

6. Blutreinigungsapparat nach einem der Ansprüche 1 bis 5, **dadurch gekennzeichnet, dass** die Blutreinigungsvorrichtung (2) einen Blutzuführungsanschluss (2a), über den das Blut in der Blutreinigungsvorrichtung (2) eingeführt werden kann, einen Blutabgabeanschluss (2b), über den das Blut aus der Blutreinigungsvorrichtung (2) abgegeben werden kann, einen Blutflussweg (2e), der sich zwischen dem Blutzuführungsanschluss (2a) und dem Blutabgabeanschluss (2b) erstreckt und durch den das Blut fließen kann, ein Dialysatzuführungsanschluss (2c), der es ermöglicht, Dialysat in die Blutreinigungsvorrichtung (2) einzuführen, ein Dialysatababeanschluss (2d), der es ermöglicht, das Dialysat aus der Blutreinigungsvorrichtung (2) abzugeben, und einen Dialysatflussweg (2f), der sich zwischen dem Dialysatzuführungsanschluss (2c) und dem Dialysatababeanschluss (2d) erstreckt und durch den das Dialysat in einer Richtung fließen kann, die einer Richtung entgegengesetzt ist, in der das Blut im Blutflussweg (2e) fließt, wobei die Vorrichtung ferner **dadurch gekennzeichnet ist, dass**, wenn die Priming-Lösung während des Primings abgelassen wird, die Blutreinigungsvorrichtung (2) so gehalten wird, dass die Blutabgabeöffnung (2b) nach oben gerichtet ist.

7. Blutreinigungsapparat nach einem der Ansprüche 2 bis 6, **dadurch gekennzeichnet, dass** er weiterhin umfasst:
eine blutkreislaufseitige Luftabscheidekammer (3), die an einer Verbindung zwischen dem venösen Blutkreislauf (1b) und der Substitutionsleitung (L3) vorgesehen ist;
eine Luftablassleitung (La), die sich von einer Oberseite der blutkreislaufseitigen Luftabscheidekammer (3) erstreckt und durch die Luft in der blutkreislaufseitigen Luftabscheidekammer (3) abgelassen werden kann; und
eine Luftabgabevorrichtung (9), die in der Lage ist, die Luft in der blutkreislaufseitigen Luftabscheidekammer (3) durch die Luftabgabeleitung nach außen abzugeben,
wobei der Apparat **dadurch gekennzeichnet ist, dass**, wenn die Priming-Lösung während des Primings zugeführt wird, das in den venösen Blutkreislauf (1b) abgegebene Gas in der blutkreislaufseitigen Luftabscheidekammer (3) gesammelt wird und das gesammelte Gas durch Aktivieren der Luftablassvorrichtung (9) durch die Luftablassleitung (La) nach außen abgelassen werden kann.

8. Blutreinigungsapparat nach Anspruch 7, **dadurch gekennzeichnet, dass** er ferner eine Flüssigkeitsniveau-Erfassungsvorrichtung (6) umfasst, die in der Lage ist, eine Flüssigkeitsoberfläche in der blutkreislaufseitigen Luftabscheidekammer (3) zu erfassen, wobei der Apparat ferner **dadurch gekennzeichnet ist, dass** die Abgabe des Gases durch die Luftablassvorrichtung (9) unter der Bedingung gestoppt wird, dass eine Flüssigkeitsoberfläche auf einem vorbestimmten Niveau durch die Flüssigkeitsniveau-Erfassungsvorrichtung (6) erfasst wird.

## Revendications

1. Appareil de purification de sang comprenant
un dispositif (2) de purification de sang comprenant des membranes (2g) de purification de sang apte à purifier du sang ;
un circuit sanguin (1) comportant un circuit sanguin artériel (1a) et un circuit sanguin veineux (1b) qui sont connectés audit dispositif (2) de purification de sang, le circuit sanguin (1) permettant de circuler le sang d'un patient de façon extracorporelle depuis une extrémité distale dudit circuit sanguin artériel (1a) vers une extrémité distale dudit circuit sanguin veineux (1b) ;
une ligne (L1) d'introduction de dialysat qui est connectée au dispositif (2) de purification de sang et qui permet que du dialysat est introduit dans le dispositif (2) de purification de sang,
une ligne (L2) de déchargement de dialysat qui est connectée au dispositif (2) de purification de sang et qui permet de décharger le liquide usagé depuis ledit dispositif (2) de purification de sang ; et
une pompe à sang (P1) qui est prévue dans le circuit sanguin artériel (1a) et qui est apte à faire circuler le sang d'un patient de façon extracorporelle depuis le circuit sanguin artériel (1a) vers le circuit sanguin veineux (1b),
ledit appareil de purification de sang étant apte à effectuer un amorçage en alimentant le circuit sanguin (1) en une solution d'amorçage lors d'une activation de la pompe à sang (P1),
lors du déchargement de dialysat pendant l'amorçage, la solution d'amorçage introduite dans le circuit sanguin (1) est permis d'être déchargée par la ligne (L2) de déchargement de dialysat,
**caractérisé en ce que**
(i) les extrémités distales du circuit sanguin artériel (1a) et du circuit sanguin veineux (1b) peuvent, toutes les deux, être connectées à une seule poche de stockage (B3) de solution d'amorçage qui est destinée à stocker la solution d'amorçage et qui est prévu pour une utilisation partagée par les deux circuits sanguins (1a, 1b), et **en ce que**, lorsque la solution d'amorçage est déchargée pendant l'amorçage, la solution d'amorçage dans la poche de stockage (B3) de solution d'amorçage est permis d'être introduite vers le circuit sanguin (1), et/ou
(ii) l'extrémité distale du circuit sanguin artériel (1a) peut être connectée à une poche de stockage (B31) de solution d'amorçage artérielle qui est destinée à stocker la solution d'amorçage tandis que l'extrémité distale du circuit sanguin veineux (1b) peut être connectée à une poche de stockage (B32) de solution d'amorçage veineuse étant compris dans l'appareil de purification de sang est destiné à stocker la solution d'amorçage, et **en ce que** lorsque la solution d'amorçage est déchargée pendant l'amorçage, la solution d'amorçage dans la poche de stockage (B31) de solution d'amorçage artérielle ou dans la poche de stockage (B32) de solution d'amorçage veineuse est permis d'être introduite vers le circuit sanguin (1).

2. Appareil de purification de sang selon la revendication 1, **caractérisé en ce qu'**il comprend en outre une ligne de substitution (L3) qui permet à une partie prédéterminée dudit circuit sanguin veineux (1b) et à ladite ligne (L1) d'introduction de dialysat de communiquer entre elles, ledit appareil étant en outre **caractérisé en ce que**, lorsque la solution d'amorçage est déchargée pendant l'amorçage, la solution d'amorçage introduite dans le circuit sanguin (1) est permis d'entrer dans la ligne (L2) de déchargement de dialysat en s'écoulant à travers la ligne de substitution (L3) et d'être déchargée.

3. Appareil de purification de sang selon la revendication 2, **caractérisé en ce que** la ligne de substitution (L3) est dotée d'une pompe de substitution (P4).

4. Appareil de purification de sang selon l'une quelconque des revendications 1 à 3, **caractérisé en ce que**, lorsque la solution d'amorçage est déchargée pendant l'amorçage, ledit appareil est capable d'effectuer de façon alternée une étape de déchargement côté extraction de sang dans laquelle la solution d'amorçage dans le circuit sanguin artériel (1a) est déchargée par la ligne (L2) de déchargement de dialysat et une étape de déchargement côté retour de sang dans laquelle la solution d'amorçage dans le circuit sanguin veineux (1b) est déchargée par la ligne (L2) de déchargement de dialysat.

5. Appareil de purification de sang selon l'une quelconque des revendications 1 à 4, **caractérisé en ce que** les extrémités distales du circuit sanguin artériel (1a) et du circuit sanguin veineux (1b) peuvent être connectées entre elles, et **en ce que** une poche de stockage (B3) de solution d'amorçage qui est destinée à stocker la solution d'amorçage peut être connectée à une ligne de bifurcation bifurquant du circuit sanguin artériel (1a) ou du circuit sanguin veineux (1b),et **en ce que**, lorsque la solution d'amorçage est déchargée pendant l'amorçage, la solution d'amorçage dans la poche de stockage (B3) de solution d'amorçage est permis d'être introduite dans le circuit sanguin (1).

6. Appareil de purification de sang selon l'une quelconque des revendications 1 à 5, **caractérisé en ce que** le dispositif (2) de purification de sang comprend un raccord (2a) d'introduction de sang qui permet que le sang est introduit dans le dispositif (2) de purification de sang, un raccord (2b) d'émission de sang qui permet que le sang est émis depuis le dispositif (2) de purification de sang, une voie (2e) de circulation de sang qui s'étend entre le raccord (2a) d'introduction de sang et le raccord (2b) d'émission de sang et qui permet la circulation du sang, un raccord (2c) d'introduction de dialysat qui permet l'introduction du dialysat dans le dispositif (2) de purification de sang, un raccord (2d) d'émission du dialysat qui permet l'émission du dialysat depuis le dispositif (2) de purification de sang, et une voie (2f) de circulation de dialysat qui s'étend entre le raccord (2c) d'introduction de dialysat et le raccord (2d) d'émission du dialysat et qui permet la circulation du dialysat dans un sens opposé au sens de circulation du sang dans la voie (2e) de circulation de sang, ledit appareil étant en outre **caractérisé en ce que**, lorsque la solution d'amorçage est déchargée pendant l'amorçage, le dispositif (2) de purification de sang est maintenu dans une position où le raccord (2b) d'émission de sang est orienté vers le haut.

7. Appareil de purification de sang selon l'une quelconque des revendications 2 à 6, **caractérisé en ce qu'**il comprend en outre :
une chambre (3) de piège à air côté circuit sanguin prévue à une jonction entre le circuit sanguin veineux (1b) et la ligne de substitution (L3) ;
une ligne (La) de déchargement d'air qui s'étend depuis un côté supérieur de la chambre (3) de piège à air côté circuit sanguin et par laquelle de l'air dans la chambre (3) de piège à air côté circuit sanguin est permis d'être déchargé ; et
un dispositif (9) de déchargement d'air capable de décharger de l'air dans la chambre (3) de piège à air côté circuit sanguin vers l'extérieur par la ligne de déchargement d'air,
ledit appareil étant caractérisé en ce, lorsque la solution d'amorçage est introduite pendant l'amorçage, du gaz déchargé vers le circuit sanguin veineux (1b) est collecté dans la chambre (3) de piège à air côté circuit sanguin, et le gaz collecté est permis d'être déchargé vers l'extérieur par la ligne (La) de déchargement d'air en activant le dispositif (9) de déchargement d'air.

8. Appareil de purification de sang selon la revendication 7, **caractérisé en ce qu'**il comprend en outre un dispositif (6) de détection de niveau du liquide qui est capable de détecter une surface du liquide dans la chambre (3) de piège à air côté circuit sanguin, ledit appareil étant en outre **caractérisé en ce que** le déchargement du gaz par le dispositif (9) de déchargement d'air est arrêté à condition qu'une surface du liquide sur un niveau prédéterminé est détectée par le dispositif (6) de détection de niveau du liquide.
